# EUROPEAN PATENT APPLICATION

(11) **EP 3 628 682 A1**
(43) Date of publication of application: **01.04.2020**
(21) Application number: 18197750.5
(22) Date of filing: 28.09.2018
(51) Int. Cl.: C07K 14/605, A61K 38/26, A61K 38/00

(54) **FORMULATIONS OF GLUCAGON-LIKE-PEPTIDE-2 (GLP-2) ANALOGUES**

(71) Applicant: Zealand Pharma A/S, 2860 Søborg (DK)
(72) Inventor: The designation of the inventor has not yet been filed
(74) Representative: Mewburn Ellis LLP

(57) **Abstract**

Stable liquid pharmaceutical formulation of glucagon-like-peptide-2 (GLP-2) analogues are disclosed and their medical use, for example in the treatment and/or prevention of stomach and bowel-related disorders and for ameliorating side effects of chemotherapy and radiation therapy. More particularly, liquid formulations of GLP-2 analogues are described that comprise a preservative and that can be stabilised for long term storage as liquids and/or as multi-dose liquid formulations.

## Description

### Field of the Invention

The present invention relates to formulations of glucagon-like-peptide-2 (GLP-2) analogues and their medical use, for example in the treatment and/or prevention of stomach and/or bowel-related disorders and for ameliorating side effects of chemotherapy and radiation therapy.

### Background of the Invention

Human GLP-2 is a 33-amino-acid peptide with the following sequence: Hy-His-Ala-Asp-Gly-Ser-Phe-Ser-Asp-Glu-Met-Asn-Thr-Ile-Leu-Asp-Asn-Leu-Ala-Ala-Arg-Asp-Phe-Ile-Asn-Trp-Leu-Ile-Gln-Thr-Lys-Ile-Thr-Asp-OH. It is derived from specific post-translational processing of proglucagon in the enteroendocrine L cells of the intestine and in specific regions of the brainstem. GLP-2 binds to a single G-protein-coupled receptor belonging to the class II glucagon secretin family.

GLP-2 has been reported to induce significant growth of the small intestinal mucosal epithelium via the stimulation of stem cell proliferation in the crypts, and by inhibition of apoptosis in the villi (Drucker et al., 1996, Proc. Natl. Acad. Sci. USA 93: 7911-7916). GLP-2 also has growth effects on the colon. Furthermore, GLP-2 inhibits gastric emptying and gastric acid secretion (Wojdemann et al., 1999, J. Clin. Endocrinol. Metab. 84: 2513-2517), enhances intestinal barrier function (Benjamin et al., 2000, Gut 47: 112-119), stimulates intestinal hexose transport via the upregulation of glucose transporters (Cheeseman, 1997, Am. J. Physiol. R1965-71), and increases intestinal blood flow (Guan et al., 2003, Gastroenterology, 125: 136-147).

It has been recognised in the art that glucagon-like peptide-2 receptor analogues have therapeutic potential for the treatment of intestinal diseases. However, the native hGLP-2, a 33 amino acid gastrointestinal peptide, is not a useful in a clinical setting due to its very short half-life in humans of around 7 minutes for full length GLP-2 [1-33] and 27 minutes for truncated GLP-2 [3-33]. In large part, the short half-life is due to degradation by the enzyme dipeptidylpeptidase IV (DPP-IV). Accordingly, there have been attempts in the art to develop GLP-2 receptor agonists with better pharmacokinetic characteristics, in particular to improve the half-life of GLP-2 molecules. By way of example, GLP-2 analogues with substitutions have been suggested such as e.g. GLP-2 analogues containing Gly substitution at position 2 ([hGly2] GLP-2, teduglutide) which increases the half-life from seven minutes (native GLP-2) to about two hours. Acylation of peptide drugs with fatty acid chains has also proven beneficial for prolonging systemic circulation as well as increasing enzymatic stability without disrupting biological potency. However, while these attempts have improved the pharmacokinetics of GLP-2 analogues, and they are sometimes described in the art as "long acting", it must be kept in mind that this is in comparison to native hGLP-2 with half-lives of the order of several hours, rather than minutes. This in turn means that the GLP-2 analogues still need to be administered to patients one or more times per day.

US 5,789,379 discloses GLP-2 analogues for administration by injection. The analogues were provided as powdered peptides and mixed with phosphate buffered saline (PBS) prior to injection at pH of 7.3-7.4 with a GLP-2 concentration of 130 mg/mL. In some instances, the GLP-2/PBS composition was mixed with gelatin to provide a depot formed from a solution of 130 mg/l GLP-2 in PBS/15% gelatin. US 5,789,379 does not disclose stable aqueous liquid formulations of GLP-2 analogues and the GLP-2 analogues are generally reconstituted from powder prior to injection.

In WO 97/39031 and US 6,184,201, the GLP-2 analogue, [Gly²]GLP-2 is disclosed. Here the alanine in position 2 has been replaced with glycine to make the peptide resistant to DPP IV cleavage. As with US 5,789,379, the GLP-2 analogue was provided as a powdered peptide and mixed with saline, PBS or 5% dextrose prior to injection, optionally adding acetic acid as a solubility enhancer.

WO 02/066511 describes GLP-2 analogues having an extended half-life *in vivo* and their use as medicaments in the treatment of gastrointestinal disorders, such as inflammatory bowel diseases. The GLP-2 analogues were stored in lyophilized form and reconstituted for administration in media, for example using saline or PBS.

WO 01/41779 describes the use of h[Gly²]GLP-2 as a pre-treatment for inhibiting chemotherapy induced apoptosis and promoting cell survival. The h[Gly²]GLP-2 is delivered by subcutaneous or intravenous injection or infusion after reconstituting the analogue in PBS.

WO 2001/049314 is directed to formulations of GLP-2 peptides and analogues thereof exhibiting superior stability following storage and/or exposure to elevated temperatures. The GLP-2 compositions comprise a GLP-2 peptide or an analogue thereof, a phosphate buffer, L-histidine, and mannitol.

WO 2006/117565 describes GLP-2 analogues which comprise one of more substitutions as compared to [hGly²]GLP-2 and which improved biological activity *in vivo* and/or improved chemical stability, e.g. as assessed in *in vitro* stability assays. In particular, GLP-2 analogues are described which have substitutions at one or more of positions 8, 16, 24 and/or 28 of the wild-type GLP-2 sequence, optionally in combination with further substitutions at position 2 and one or more of positions 3, 5, 7, 10 and 11, and/or a deletion of one or more of amino acids 31 to 33. These substitutions may also be combined with the addition of a N-terminal or C-terminal stabilizing peptide sequence. The daily or twice daily administration of these GLP-2 analogues is also described. Among the molecules disclosed in WO 2006/117565 is glepaglutide (ZP1848) which has been designed to be stable in liquid formulations, and is typically administered by daily dosing using an injection pen.

WO 2005/049061 relates to formulations of GLP-1 agonists that include propylene glycol to reduce clogging of devices used to administer the peptide and for reducing deposits of the peptide on production equipment.

It remains a problem in this area to improve the formulation of GLP-2 analogues, in particular to provide stable liquid formulations that are capable of long term storage without undue levels of physical degradation of the active monomeric form of the peptide occurring. The stability of formulations of GLP-2 analogues is a particular problem in situations where it is desirable to include a preservative in the formulation, either for regulatory reasons and/or where the formulation is a multi-dose formulation, as preservatives tend to destabilise formulations of peptide drugs in an unpredictable manner.

### Summary of the Invention

Broadly, the present invention is based on studies reported in the examples that led to surprising findings relating to liquid formulations of GLP-2 analogues comprising a preservative can be stabilised for long term storage as liquids and/or as multi-dose liquid formulations. Broadly, the present inventors found that reducing the pH of the formulation to below 7.0, e.g. to a pH between about pH 5.0 and about 6.8, was able to offset the effect that the addition of a preservative such as meta-cresol has on the physical stability of the formulation. Alternatively or additionally, the present inventors found that the addition of an excipient such as propylene glycol was similarly able to offset the effect that the addition of a preservative, such as meta-cresol, has on the physical stability of the formulation. This opens up the possibility of being able to deliver the GLP-2 analogues as multi-dose formulations. This further opens up the possibility of providing the formulations of the GLP-2 analogue in the form of a drug delivery device, and particularly multi-dose delivery devices, such as a pre-filled syringe, an adjustable dose auto-injector, a disposable auto-injector, a wearable injector or an infusion pump, thereby providing patients with a ready-to-use formulation in a simpler, safer and more patient-friendly device.

Furthermore, the present inventors found that the inclusion of the preservative in the GLP-2 analogue formulations did not have any significant adverse effect on the chemical stability of the peptide. Thus, the components of the formulation and their amounts provide a formulation with at least 90% content of the GLP-2 analogue and with less than 10% of chemical degradation products at storage for at least 18 months at 2-8°C.

Accordingly, in a first aspect, the present invention provides a stable liquid pharmaceutical multi-dose formulation, the formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
(a) the GLP-2 analogue at a concentration of about 1 mg/mL to about 30 mg/mL;
(b) meta-cresol as a preservative at a concentration of 1.0 to 5.0 mg/mL;
(c) a histidine buffer at a concentration of about 5 mM to about 50 mM;
(d) a non-ionic tonicity modifier selected from the group consisting of mannitol, sucrose, glycerol and sorbitol at a concentration of about 20 mM to about 360 mM;
(e) a pH of about 5.0 to about 6.8.

In a further aspect, the present invention provides a stable liquid pharmaceutical multi-dose formulation, the formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
(a) the GLP-2 analogue at a concentration of about 1 mg/mL to about 30 mg/mL;
(b) meta-cresol as a preservative at a concentration of 1.0 to 5.0 mg/mL;
(c) a histidine buffer at a concentration of about 5 mM to about 50 mM;
(d) a non-ionic tonicity modifier selected from the group consisting of mannitol, sucrose, glycerol and sorbitol at a concentration of about 20 mM to about 360 mM;
(e) a pH of about 5.5 to about 7.0; and
(f) propylene glycol at a concentration of 20 mM to 300 mM.

In some embodiments, the formulations of the present invention comprises propylene glycol at a concentration of 50 mM to 300 mM, more preferably at a concentration of 50 mM to 200 mM, more preferably at a concentration of 50 mM to 150 mM, more preferably at a concentration of 150 mM to 200 mM.

In a further aspect, the present invention provides an article of manufacture or a kit comprising a container holding the stable pharmaceutical formulation of the present invention.

In a further aspect, the present invention provides a delivery device containing a liquid formulation comprising a GLP-2 analogue of present invention.

In a further aspect, the present invention provides a formulation of the glucagon-like peptide 2 (GLP-2) analogue of the present invention for use in therapy.

In a further aspect, the present invention provides a formulation of the glucagon-like peptide 2 (GLP-2) analogue of the present invention for use in a method for the treatment and/or prevention of a stomach and/or bowel-related disorder in a human patient.

In a further aspect, the present invention provides a process for producing a stable liquid multi-dose pharmaceutical formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
wherein the process comprising formulating (a) the GLP-2 analogue at a concentration of about 1 mg/mL to about 30 mg/mL; (b) meta-cresol as a preservative at a concentration of 1.0 to 5.0 mg/mL; (c) a histidine buffer at a concentration of about 5 mM to about 50 mM; (d) a non-ionic tonicity modifier selected from the group consisting of mannitol, sucrose, glycerol and sorbitol at a concentration of about 20 mM to about 360 mM; at a pH of about 5.0 to about 6.8.

In a further aspect, the present invention provides a process for producing a stable liquid multi-dose pharmaceutical formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
wherein the process comprising formulating (a) the GLP-2 analogue at a concentration of about 1 mg/mL to about 30 mg/mL; (b) meta-cresol as a preservative at a concentration of 1.0 to 5.0 mg/mL; (c) a histidine buffer at a concentration of about 5 mM to about 50 mM; (d) a non-ionic tonicity modifier selected from the group consisting of mannitol, sucrose, glycerol and sorbitol at a concentration of about 20 mM to about 360 mM; (e) propylene glycol at a concentration of 20 mM to 300 mM; at a pH of about 5.5 to about 7.0.

In a further aspect, the present invention provides the use of a formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
for providing a multi-dose liquid pharmaceutical formulation which is stable for 18 months when stored at 2-8°C, wherein the formulation comprises:
(a) the GLP-2 analogue at a concentration of about 1 mg/mL to about 30 mg/mL;
(b) meta-cresol as a preservative at a concentration of 1.0 to 5.0 mg/mL;
(c) a histidine buffer at a concentration of about 5 mM to about 50 mM;
(d) a non-ionic tonicity modifier selected from the group consisting of mannitol, sucrose, glycerol and sorbitol at a concentration of about 20 mM to about 360 mM;
(e) a pH of about 5.0 to about 6.8.

In a further aspect, the present invention provides the use of a formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
for providing a multi-dose liquid pharmaceutical formulation which is stable for 18 months when stored at 2-8°C, wherein the formulation comprises:
wherein the formulation comprises:
(a) the GLP-2 analogue at a concentration of about 1 mg/mL to about 30 mg/mL;
(b) meta-cresol as a preservative at a concentration of 1.0 to 5.0 mg/mL;
(c) a histidine buffer at a concentration of about 5 mM to about 50 mM;
(d) a non-ionic tonicity modifier selected from the group consisting of mannitol, sucrose, glycerol and sorbitol at a concentration of about 20 mM to about 360 mM;
(e) a pH of about 5.5 to about 7.0; and
(f) propylene glycol at a concentration of 20 mM to 300 mM.

In a further aspect, the present invention relates to a stable liquid multi-dose pharmaceutical formulation, the formulation comprising a glucagon-like peptide 2 (GLP-2) analogue, wherein the GLP-2 analogue is represented by the formula: 
R¹- His-Gly-Glu-Gly-X5-Phe-Ser-Ser-Glu-Leu-X11-Thr-Ile-Leu-Asp-Ala-Leu-Ala-Ala-
Arg-Asp-Phe-Ile-Ala-Trp-Leu-Ile-Ala-Thr-Lys-Ile-Thr-Asp-Z²-R²
 wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z² is a peptide sequence of 6 amino acid units of Lys;
or a pharmaceutically acceptable salt or derivative thereof.

In this aspect of the present invention, the glucagon-like peptide 2 (GLP-2) analogue may be used for the treatment and/or prevention of stomach and/or bowel-related disorders such as ulcers, digestion disorders, malabsorption syndromes, short-gut syndrome, cul-de-sac syndrome, inflammatory bowel disease, celiac sprue (for example arising from gluten induced enteropathy or celiac disease), tropical sprue, hypogammaglobulinemic sprue, enteritis, regional enteritis (Crohn's disease), ulcerative colitis, small intestine damage or short bowel syndrome (SBS). Alternatively or additionally, the glucagon-like peptide 2 (GLP-2) analogue may be used for the treatment and/or prevention of stomach and/or bowel-related disorders such radiation enteritis, infectious or post-infectious enteritis, or small intestinal damage due to toxic or other chemotherapeutic agents. In this case, treatment with the GLP-2 analogue may optionally be combined with one or more anti-cancer therapies, and may therefore comprise administering one or more chemotherapeutic agent(s) to the patient or treating the patient with radiation therapy.

In some embodiments of the present invention, in the above formula, X5 is Thr and/or X11 is Ala. Examples of these glucagon-like peptide 2 (GLP-2) analogues include:
ZP1848 H-HGEGTFSSELATILDALAARDFIAWLIATKITDKKKKKK-NH₂ (SEQ ID NO: 1) ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-NH₂ (SEQ ID NO: 5); or
ZP2530 H-HGEGTFSSELATILDALAARDFIAWLIATKITD-OH (SEQ ID NO: 6).

In some embodiments of the present invention, in the above formula X5 is Ser and/or X11 is Ser. Examples of these glucagon-like peptide 2 (GLP-2) analogues include:
ZP1846 H-HGEGSFSSELSTILDALAARDFIAWLIATKITDKKKKKK-NH₂ (SEQ ID NO: 7);
ZP1855 H-HGEGSFSSELSTILDALAARDFIAWLIATKITD-NH₂ (SEQ ID NO: 8); or
ZP2242 H-HGEGSFSSELSTILDALAARDFIAWLIATKITDK-OH (SEQ ID NO: 9).

Embodiments of the present invention will now be described by way of example and not limitation. However, various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

### Detailed Description of the Invention

### Definitions

Unless specified otherwise, the following definitions are provided for specific terms, which are used in the above written description.

Throughout the description and claims the conventional one-letter and three-letter codes for natural amino acids are used. All amino acid residues in peptides of the invention are preferably of the L-configuration, However, D-configuration amino acids may also be present.

Preferred compounds of the present invention have at least one GLP-2 biological activity, in particular in causing growth of the intestine. This can be assessed in *in vivo* assays, for example as described in the examples of (e.g.) WO 2006/117565, in which the mass of the intestine, or a portion thereof is determined after a test animal has been treated or exposed to a GLP-2 analogue.

The liquid formulations according to the present invention are preferably an isosmotic liquid formulation. "Isosmotic" means that the formulations of the present invention have the same or a similar osmotic pressure with bodily fluids. Preferably, the formulations of the present invention have an osmolality of about 300 ± 60 mOsm as measured by an osmometer.

The formulations of the present invention generally contain the GLP-2 analogue at a concentration of about 1 mg/mL to about 30 mg/mL, more preferably at a concentration of about 1 mg/mL to about 20 mg/mL, more preferably at a concentration of about 1 mg/mL to about 15 mg/mL, and more preferably at a concentration of about 2 mg/mL or about 10 mg/mL, and most preferably at a concentration of about 2 mg/mL or about 20 mg/mL. The amount of GLP-2 analogue and its degradation products may be determined by HPLC using techniques well known to the skilled person.

In some embodiments, the formulation of the present invention may be used in a once or twice daily dosage regime. In some cases, the formulation of the present invention may be used in a once or twice weekly dosage regime. Alternatively or additionally, the dosing regime of the GLP-2 analogues of the present invention may comprise a plurality or course of doses separated in time by 2 days, 2.5 days, 3 days, 3.5 days, 4 days, 5 days, 6 days, 7 days, 8 days, 9 days, 10 days, 11 days or 12 days. In a preferred embodiment, the doses are separated in time by 3 days, 3.5 days, 4 days, 5 days, 6 days, 7 days or 8 days. In a preferred embodiment, doses are separated in time by 3 days, 3.5 days, 4 days or 7 days. As will be appreciated in the art, the time between doses may be varied to some extent so that each and every doses is not separated by precisely the same time. This will often be directed under the discretion of the physician. Thus, doses may be separated in time by a clinically acceptable range of times, e.g. from about 2 days to about 10 days, or from about 3 or 4 days to about 7 or 8 days.

The formulations of the present invention are stable liquid pharmaceutical formulations of GLP-2 analogues. A "stable" formulation is one in which the peptide therein essentially retains its physical stability and/or chemical stability and/or biological activity upon storage. Preferably, the formulation essentially retains its physical and chemical stability, as well as its biological activity upon storage. The storage period is generally selected based on the intended shelf-life of the formulation. The formulations of the present invention are provided as stable liquid formulations, e.g. stable aqueous liquid formulations. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993), for example. In the present invention, "stable" formulations include formulations in which at least 80%, more preferably at least 90%, more preferably at least 95%, more preferably at least 96%, more preferably at least 97%, more preferably at least 98%, and most preferably at least 99% of the GLP-2 analogue is active in the formulation after it has been stored at 2-8°C for at least 18 months.

Stability can be measured at a selected temperature for a selected time period, for example using elevated temperature to reduce the period over which a formulation is tested. Generally, storage at a temperature between 2 to 8°C denotes storage under normal refrigerated conditions. In certain embodiments, the formulation is stable under such conditions for at least 12 months, more preferably at least 18 months, more preferably at least 24 months. Stability can be evaluated qualitatively and/or quantitatively in a variety of different ways, including evaluation of degradation products (e.g. using HPLC and/or LC-MS), aggregate formation (e.g. using size exclusion chromatography, by measuring turbidity, and/or by visual inspection); by assessing charge heterogeneity using cation exchange chromatography, image capillary isoelectric focusing (icIEF) or capillary zone electrophoresis; amino-terminal or carboxy-terminal sequence analysis; mass spectrometric analysis; SDS-PAGE analysis to compare reduced and intact antibody; peptide map (for example tryptic or LYS-C) analysis; evaluating biological activity or antigen binding function of the antibody; etc. Instability may involve any one or more of: aggregation, deamidation (e.g. Asn deamidation), oxidation (e.g. Met oxidation), isomerization (e.g. Asp isomeriation), clipping/hydrolysis/fragmentation (e.g. hinge region fragmentation), succinimide formation, unpaired cysteine(s), N-terminal extension, C-terminal processing, glycosylation differences, etc.

A peptide "retains its physical stability" in a pharmaceutical formulation if it shows no sign (or very little sign) of aggregation, precipitation and/or denaturation upon e.g. visual examination of colour and/or clarity, or as measured by UV light scattering, dynamic light scattering, circular dichroism, or by size exclusion chromatography and is considered to still retain its biological activity.

A peptide "retains its chemical stability" in a pharmaceutical formulation, if the chemical stability at a given time is such that the peptide is considered to still retain its biological activity as defined below. Chemical stability can be assessed by detecting and quantifying chemically altered forms of the peptide. Chemical alteration may involve isomerization, oxidation, size modification (e.g. clipping) which can be evaluated using HPLC or size exclusion chromatography, SDS-PAGE and/or mass spectrometry, for example. Other types of chemical alteration include charge alteration n (e.g. occurring as a result of deamidation) which can be evaluated by HPLC or ion-exchange chromatography or iclEF, for example.

### GLP-2 analogues

The GLP-2 analogues present in the formulations of the present invention have one or more amino acid substitutions, deletions, inversions, or additions compared with native GLP-2 and as defined above. This definition also includes the synonym terms GLP-2 mimetics and/or GLP-2 agonists. Further, the analogue of the present invention may additionally have chemical modification of one or more of its amino acid side groups, α-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the des-amino, N-lower alkyl, N-di-lower alkyl, and N-acyl modifications. Modifications of the terminal carboxy group include, without limitation, the amide, lower alkyl amide, dialkyl amide, and lower alkyl ester modifications. Preferably herein lower alkyl is C₁-C₄ alkyl. Furthermore, one or more side groups, or terminal groups, may be protected by protective groups known to the ordinarily-skilled peptide chemist. The α-carbon of an amino acid may be mono- or di-methylated.

In some aspects, the liquid formulations of the present invention employ a glucagon-like peptide 2 (GLP-2) analogue represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof.

In some embodiments of the present invention, in the above formula, X5 is Thr and/or X11 is Ala. Examples of these glucagon-like peptide 2 (GLP-2) analogues include:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-NH₂ (SEQ ID NO: 5); or
ZP2530 H-HGEGTFSSELATILDALAARDFIAWLIATKITD-OH (SEQ ID NO: 6).

In an embodiment of the present invention, the glucagon-like peptide 2 (GLP-2) analogue is ZP1848 H-HGEGTFSSELATILDALAARDFIAWLIATKITDKKKKKK-NH₂ (SEQ ID NO: 1).

In some embodiments of the present invention, in the above formula X5 is Ser and/or X11 is Ser. Examples of these glucagon-like peptide 2 (GLP-2) analogues include:
ZP1846 H-HGEGSFSSELSTILDALAARDFIAWLIATKITDKKKKKK-NH₂ (SEQ ID NO: 7);
ZP1855 H-HGEGSFSSELSTILDALAARDFIAWLIATKITD-NH₂ (SEQ ID NO: 8); or
ZP2242 H-HGEGSFSSELSTILDALAARDFIAWLIATKITDK-OH (SEQ ID NO: 9).

In an embodiment of the present invention, the glucagon-like peptide 2 (GLP-2) analogue is ZP1846 H-HGEGSFSSELSTILDALAARDFIAWLIATKITDKKKKKK-NH₂ (SEQ ID NO: 7).

It should be understood that the peptides (drug substance) of the invention might also be provided in the form of a salt or other derivative. Salts include pharmaceutically acceptable salts, such as acid addition salts and basic salts. Examples of acid addition salts include hydrochloride salts, citrate salts, chloride salts and acetate salts. Preferably, the salt is acetate. Examples of basic salts include salts where the cation is selected from alkali metals, such as sodium and potassium, alkaline earth metals, such as calcium, and ammonium ions ⁺N (R³)₃(R⁴), where R³ and R⁴ independently designates optionally substituted C₁₋₆-alkyl, optionally substituted C₂₋₆-alkenyl, optionally substituted aryl, or optionally substituted heteroaryl. Other examples of pharmaceutically acceptable salts are described in "Remington's Pharmaceutical Sciences",17th edition. Ed. Alfonso R. Gennaro (Ed.), Mark Publishing Company, Easton, PA, U.S.A., 1985 and more recent editions, and in the Encyclopaedia of Pharmaceutical Technology.

In preferred embodiments, the GLP-2 analogue of the invention is selected from the group consisting of ZP1848-acetate, ZP2949-acetate, ZP2711-acetate, ZP2469-acetate, ZP1857-acetate, ZP2530-acetate, ZP1846-acetate, ZP1855-acetate and ZP2242-acetate. In the present context, the term "ZP1848-acetate" refers to the ZP1848 molecule is in the form of an acetate salt.

In a preferred embodiment, the GLP-2 analogue is ZP1848-acetate. HGEGTFSSELATILDALAARDFIAWLIATKITDKKKKKK-NH₂ acetate (SEQ ID NO: 1).

Other derivatives of the GLP-2 analogues of the invention include coordination complexes with metal ions such as Mn²⁺ and Zn²⁺, esters such as *in vivo* hydrolysable esters, free acids or bases, hydrates, prodrugs or lipids. Esters can be formed between hydroxyl or carboxylic acid groups present in the compound and an appropriate carboxylic acid or alcohol reaction partner, using techniques well known in the art. Derivatives which as prodrugs of the compounds are convertible *in vivo* or *in vitro* into one of the parent compounds. Typically, at least one of the biological activities of compound will be reduced in the prodrug form of the compound, and can be activated by conversion of the prodrug to release the compound or a metabolite of it. Examples of prodrugs include the use of protecting groups which may be removed *in situ* releasing active compound or serve to inhibit clearance of the drug *in vivo.*

Z¹ and Z² are independently present and/or absent or a peptide sequence of 1-6 amino acid units of Lys, i.e. 1, 2, 3, 4, 5 or 6 Lys residues. The Lys residues may have either D- or L-configuration, but have an L-configuration. Particularly preferred sequences Z are sequences of four, five or six consecutive lysine residues, and particularly six consecutive lysine residues. Exemplary sequences Z are shown in WO 01/04156. In certain embodiments, Z¹ is absent. In such cases, Z² may be either present or absent.

### Formulations of the GLP-2 analogues

The formulation of the GLP-2 analogues is a ready-to-use formulation. The term "ready-to-use" as used herein, refers to a formulation that does not require constitution or dilution with a prescribed amount of diluent, e.g., water for injection or other suitable diluent, before use by the designated route of administration.

As described herein, the liquid formulations of the GLP-2 analogues of the present invention include a buffer, a non-ionic tonicity modifier and arginine q.s. to provide the pH of the final formulation. In accordance with normal pharmaceutical practice, the formulations of the present invention are sterile and/or free from reducing agent. In some cases, the liquid formulations of the present invention are aqueous, liquid formulations.

The term "buffer" as used herein denotes a pharmaceutically acceptable excipient which stabilizes the pH of a pharmaceutical formulation. Suitable buffers are well known in the art and can be found in the literature. In preferred embodiments, the buffer is selected from the group consisting of a histidine buffer, acetate buffer and mesylate buffer as these buffers provided stable formulations in which the GLP-2 analogues dissolved and did not become viscous, cloudy or precipitate the peptide drug. In preferred embodiments, the buffer is a histidine buffer, e.g. L-histidine. Generally, the buffer will be present at a concentration of about 5 mM to about 50 mM, more preferably at a concentration of about 5 mM to about 25 mM, and most preferably at a concentration of about 15 mM. Preferably, the buffer is not a phosphate buffer, a citrate buffer, citrate/Tris buffer and/or succinate buffer.

The term "tonicity modifier" as used herein denotes pharmaceutically acceptable tonicity agents that are used to modulate the tonicity of the formulation. The formulations of the present invention are preferably isosmotic, that is they have an osmotic pressure that is substantially the same as human blood serum. The tonicity modifiers used in the formulations are preferably non-ionic tonicity modifiers and are preferably selected from the group consisting of mannitol, sucrose, glycerol and sorbitol. A preferred non-ionic tonicity modified is mannitol, e.g. D-mannitol. The concentration of the tonicity modifier will be dependent on the concentration of other components of the formulation, especially where the formulation is intended to be isosmotic. Typically, the non-ionic tonicity modifier will be employed at a concentration of about 90 mM to about 360 mM, more preferably at a concentration of about 150 mM to about 250 mM, and most preferably at a concentration of about 230 mM.

Generally, the components and amounts of the liquid formulations of the present invention are chosen to provide a formulation with a pH of about 5.0 to about 6.8, in aspects of the present invention where a reduction in pH is used to compensate for the addition of preservative to the formulation. In an preferred embodiment, where a reduction in pH is used to compensate for the addition of preservative to the formulation, the formulations may have a pH between about 6.2 and about 6.8. Alternatively or additionally, in aspects of the present invention in which propylene glycol is present the formulations may have a pH between about 5.5 and about 7.0, such as about 6.0 and about 7.0, about 6.8 and about 7.0.

In a further embodiment, the liquid formulations of the present invention comprises:
(a) the GLP-2 analogue at a concentration of about 2 mg/mL to about 20 mg/mL;
(b) meta-cresol as a preservative at a concentration of 1.0 to 5.0 mg/mL;
(c) a histidine buffer at a concentration of about 5 mM to about 50 mM;
(d) mannitol as a non-ionic tonicity modifier at a concentration of about 20 mM to about 360 mM;
(e) a pH of about 5.5 to about 7.0; and
(f) propylene glycol at a concentration of 20 mM to 300 mM.

In a further embodiment, the liquid formulations of the present invention comprises:
(a) the GLP-2 analogue at a concentration of about 10 mg/mL;
(b) a meta-cresol as a preservative at a concentration of 3.0 to 4.0 mg/mL;
(c) a histidine buffer at a concentration of about 10 mM to about 20 mM;
(d) mannitol as a non-ionic tonicity modifier at a concentration of about 50 mM to about 230 mM; and
(e) a pH of about 5.0 to about 7.0; and
(f) propylene glycol at a concentration of 50 mM to 200 mM.

In a further embodiment, the liquid formulations of the present invention consists of the GLP-2 analogue at a concentration of about 10 mg/mL; meta-cresol as a preservative at a concentration of 3.0 to 4.0 mg/mL; histidine buffer at a concentration of about 10 mM to about 20 mM; mannitol as a non-ionic tonicity modifier at a concentration of about 50 mM to about 230 mM; and propylene glycol at a concentration of 50 mM to 200 mM, the formulation having a pH of about 5.5 to about 7.0.

In a further embodiment, the liquid formulations of the present invention comprises:
(a) the GLP-2 analogue at a concentration of about 1 mg/mL to about 15 mg/mL;
(b) meta-cresol as a preservative at a concentration of 1.0 to 5.0 mg/mL;
(c) a histidine buffer at a concentration of about 5 mM to about 50 mM;
(d) mannitol as a non-ionic tonicity modifier at a concentration of about 20 mM to about 360 mM; and
(e) has a pH of about 6.0 to about 6.8.

In a further embodiment, the liquid formulations of the present invention comprises:
(a) the GLP-2 analogue at a concentration of about 2 mg/mL or about 10 mg/mL;
(b) a meta-cresol as a preservative at a concentration of 3.0 to 4.0 mg/mL;
(c) a histidine buffer at a concentration of about 10 mM to about 20 mM;
(d) mannitol as a non-ionic tonicity modifier at a concentration of about 200 mM to about 250 mM; and
(e) has a pH of about 6.0 to about 6.8.

In a further embodiment, the liquid formulations of the present invention comprises the GLP-2 analogue at a concentration of about 2 mg/mL, about 10 mg/mL or about 10 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.0 to about 6.8.

In a further embodiment, the liquid formulations of the present invention comprises the GLP-2 analogue at a concentration of about 2 mg/mL, about 10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulations of the present invention comprises ZP1848-acetate or H-HGEGTFSSELATILDALAARDFIAWLIATKITDKKKKKK-NH2 acetate (SEQ ID NO: 1). at a concentration of about 2 mg/mL, about 10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulations of the present invention comprises ZP1846 H-HGEGSFSSELSTILDALAARDFIAWLIATKITDKKKKKK-NH2 (SEQ ID NO: 7);
at a concentration of about 2 mg/mL, about 10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulations of the present invention consists of the GLP-2 analogue at a concentration of about 2 mg/mL or about 10 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.0 to about 6.8.

In a further embodiment, the liquid formulations of the present invention consists of the GLP-2 analogue at a concentration of about 2 mg/mL, about10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulations of the present invention consists of ZP1848-acetate or H-HGEGTFSSELATILDALAARDFIAWLIATKITDKKKKKK-NH2 acetate (SEQ ID NO: 1). at a concentration of about 2 mg/mL, about 10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulations of the present invention consists of ZP1846 H-HGEGSFSSELSTILDALAARDFIAWLIATKITDKKKKKK-NH2 (SEQ ID NO: 7);
at a concentration of about 2 mg/mL, about 10 mg/mL or about 20 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.2 to about 6.8.

In a further embodiment, the liquid formulation is selected from the group consisting of an aqueous liquid formulation, a liquid formulation in various hydrophilic or hydrophobic solvents, an emulsion and a liquid suspension. In a preferred embodiment, the liquid formulation is an aqueous liquid formulation. In some cases, the liquid formulations of the present invention are non-aqueous, liquid formulations.

Preferably, the glucagon-like peptide 2 (GLP-2) analogue are administered to patients parenterally, preferably by injection, most typically by subcutaneous injection, intramuscular injection, intravenous injection or intraperitoneal injection. Administration by subcutaneous injection is preferred. The injection may be carried out by a physician, nurse or other healthcare professional, or may be self-administered by the patient. As set out herein, in some aspects, the formulations of the present invention have a viscosity that facilitates loading of the formulation into a pre-filled syringe, an injection pen or other injector device. This may have the advantage of pre-determining the dose of the formulation administered to the patient, e.g. without the need for measurement from a multi-dose vial. Accordingly, in other aspects, the present invention provides an article of manufacture or a kit comprising a container holding the stable, such as e.g. an aqueous stable pharmaceutical formulation of the GLP-2 analogue according to the present invention or a pre-filled syringe or injector device or injector pen containing an aqueous liquid formulation comprising the GLP-2 analogue according to the present invention.

### Medical Conditions

The GLP-2 analogue formulations of the present invention are useful as a pharmaceutical agent for preventing or treating an individual suffering from gastro-intestinal disorders, including the upper gastrointestinal tract of the oesophagus by administering an effective amount of a GLP-2 analogue, or a salt thereof as described herein. The stomach and intestinal-related disorders include ulcers of any aetiology (e.g., peptic ulcers, drug-induced ulcers, ulcers related to infections or other pathogens), digestion disorders, malabsorption syndromes, short-bowel syndrome, cul-de-sac syndrome, inflammatory bowel disease, celiac sprue (for example arising from gluten induced enteropathy or celiac disease), tropical sprue, hypogammaglobulinemic sprue, enteritis, ulcerative colitis, small intestine damage, and chemotherapy induced diarrhoea/mucositis (CID).

As mentioned above, in general individuals who would benefit from increased small intestinal mass and consequent and/or maintenance of normal small intestine mucosal structure and function are candidates for treatment with the present GLP-2 analogues. Particular conditions that may be treated with GLP-2 analogue include the various forms of sprue including celiac sprue which results from a toxic reaction to alpha-gliadin from heat and may be a result of gluten-induced enteropathy or celiac disease, and is marked by a significant loss of villae of the small bowel; tropical sprue which results from infection and is marked by partial flattening of the villae; hypogammaglobulinemic sprue which is observed commonly in patients with common variable immunodeficiency or hypogammaglobulinemia and is marked by significant decrease in villus height. The therapeutic efficacy of the GLP-2 analogue treatment may be monitored by enteric biopsy to examine the villus morphology, by biochemical assessment of nutrient absorption, by patient weight gain, or by amelioration of the symptoms associated with these conditions.

Another particular condition which may be treated with the GLP-2 analogues of the invention, or for which the GLP-2 analogues may be useful therapeutically and/or prophylactically is short bowl syndrome (SBS), also known as short gut syndrome or simply short gut, which results from surgical resection, congenital defect or disease-associated loss of absorption in the bowel in which patients are subsequently unable to maintain fluid, electrolyte, and nutrient balances on a conventional diet. Despite an adaptation that occurs generally in the two years after resection, SBS patients have reduced dietary uptake and fluid loss.

Other conditions that may be treated with the GLP-2 analogues of the invention, or for which the GLP-2 analogues may be useful prophylactically, include in addition to the above mentioned radiation enteritis, infectious or post-infectious enteritis, and small intestinal damage due to cancer-chemotherapeutic or toxic agents.

The GLP-2 analogues may also be used for the treatment of malnutrition, for example cachexia and anorexia.

A particular embodiment of the invention is concerned with using the present peptides for the prevention and/or treatment of intestinal damage and dysfunction. Such damage and dysfunction is a well-known side effect of cancer-chemotherapy treatment. Chemotherapy administration is frequently associated with unwanted side effects related to the gastronintestinal system such as mucositis, diarrhoea, bacterial translocation, malabsorption, abdominal cramping, gastrointestinal bleeding and vomiting. These side effects are clinical consequences of the structural and functional damage of the intestinal epithelium and frequently make it necessary to decrease the dose and frequency of chemotherapy.

Administration of the present GLP-2 peptide analogues may enhance trophic effect in the intestinal crypts and rapidly provide new cells to replace the damaged intestinal epithelium following chemotherapy. The ultimate goal achieved by administering the present peptides is to reduce the morbidity related to gastrointestinal damage of patients undergoing chemotherapy treatment while creating the most optimal chemotherapy regime for the treatment of cancer. Concomitant prophylactic or therapeutic treatment may be provided in accordance with the present invention to patients undergoing or about to undergo radiation therapy.

The stem cells of the small intestinal mucosa are particularly susceptible to the cytotoxic effects of chemotherapy due to their rapid rate of proliferation (Keefe et al., Gut, 47: 632-7, 2000). Chemotherapy-induced damage to the small intestinal mucosa is clinically often referred to as gastrointestinal mucositis and is characterized by absorptive and barrier impairments of the small intestine. For example, it has been shown that, the broadly used chemotherapeutic agents, 5-FU, irinotecan and methothrexate increase apoptosis leading to villus atrophy and crypt hypoplasia in the small intestine of rodents (Keefe et al., Gut 47: 632-7, 2000; Gibson et al., J Gastroenterol. Hepatol. Sep;18(9):1095-1100, 2003; Tamaki et al., J. Int. Med. Res. 31(1):6-16, 2003). Chemotherapeutic agents have been shown to increase apoptosis in intestinal crypts at 24 hours after administration and subsequently to decrease villus area, crypt length, mitotic count per crypt, and enterocyte height three days after chemotherapy in humans (Keefe et al., Gut, 47: 632-7, 2000). Thus, structural changes within the small intestine directly lead to intestinal dysfunction and in some cases diarrhoea.

Gastrointestinal mucositis after cancer chemotherapy is an increasing problem that is essentially untreatable once established, although it gradually remits. Studies conducted with the commonly used cytostatic cancer drugs 5-FU and irinotecan have demonstrated that effective chemotherapy with these drugs predominantly affects structural integrity and function of the small intestine while the colon is less sensitive and mainly responds with increased mucus formation (Gibson et al., J. Gastroenterol. Hepatol. Sep;18(9):1095-1100, 2003; Tamaki et al., J Int. Med. Res. 31(1):6-16, 2003).

The formulations of the present invention comprising GLP-2 analogues may be useful in the prevention and/or treatment of gastrointestinal injury and side effects of chemotherapeutic agents. This potentially important therapeutic application may apply to currently used chemotherapeutic agents such as but not limited to: 5-FU, Altretamine, Bleomycin, Busulfan, Capecitabine, Carboplatin, Carmustine, Chlorambucil, Cisplatin, Cladribine, Crisantaspase, Cyclophosphamide, Cytarabine, Dacarbazine, Dactinomycin, Daunorubicin, Docetaxel, Doxorubicin, Epirubicin, Etoposide, Fludarabine, Fluorouracil, Gemcitabine, Hydroxycarbamide, Idarubicin, Ifosfamide, Irinotecan, Liposomal doxorubicin, Leucovorin, Lomustine, Melphalan, Mercaptopurine, Mesna, Methotrexate, Mitomycin, Mitoxantrone, Oxaliplatin, Paclitaxel, Pemetrexed, Pentostatin, Procarbazine, Raltitrexed, Streptozocin, Tegafur-uracil, Temozolomide, Thiotepa, Tioguanine/Thioguanine, Topotecan, Treosulfan, Vinblastine, Vincristine, Vindesine, Vinorelbine, Bleomycin, Busulfan, Capecitabine, Carboplatin, Carmustine, Chlorambucil, Cisplatin, Cladribine, Crisantaspase, Cyclophosphamide, Cytarabine, Dacarbazine, Dactinomycin, Daunorubicin, Docetaxel, Doxorubicin, Epirubicin, Etoposide, Fludarabine, Fluorouracil, Gemcitabine, Hydroxycarbamide, Idarubicin, Ifosfamide, Irinotecan, Liposomal doxorubicin, Leucovorin, Lomustine, Melphalan, Mercaptopurine, Methotrexate, Mitomycin, Mitoxantrone, Oxaliplatin, Paclitaxel, Pemetrexed, Pentostatin, Procarbazine, Raltitrexed, Streptozocin, Tegafur-uracil, Temozolomide, Thiotepa, Tioguanine/Thioguanine, Topotecan, Treosulfan, Vinblastine, Vincristine, Vindesine, and Vinorelbine.

### Delivery of the Formulations

In some aspects, the present invention relates to a ready-to-use formulation of GLP-2 analogues, intended for parenteral administration, and suitable for use in e.g. vials, pre-filled syringes, infusion pumps, wearable injectors, disposable auto-injectors or adjustable dose auto-injectors.

### Examples

The following examples are provided to illustrate preferred aspects of the invention and are not intended to limit the scope of the invention. The GLP-2 analogues administered according to the dosage regimes described herein can be made according to the methods such as solid phase peptide synthesis described in WO 2006/117565, the content of which is expressly incorporated by reference in its entirety.

### Example 1. Synthesis of ZP1848 and similar GLP-2 analogues

ZP1848 peptide was synthesized using a Solid Phase Peptide Synthesis (SPPS) approach and standard Fmoc coupling methodologies. After completed synthesis, the peptide sequence was de-protected and cleaved from the solid support, and the crude peptide was purified using preparative reverse phase HPLC. The peptide was converted to the acetate salt form or other acceptable salts and lyophilised to provide the final ZP1848 drug substance.

This synthesis and purification protocol may be adapted for making other GLP-2 analogues used in the formulations of the present invention.

### Example 2: Effect of adding preservative to GLP-2 analogue formulations

Excipient stock solutions of L-histidine (200 mM), mannitol (500 mM), m-cresol (1034 mg/mL)) and propylene glycol (1036 mg/ml) were prepared. Excipient solutions were mixed according to the desired composition (see Table 1) and stock solution of ZP1848 (approx. 40 mg/mL) was added. The volume was adjusted to 90% of final volume, pH was adjusted with acetic acid (200 mM) and/or L-arginine (200 to 250 mM), m-cresol was added, and the volume was adjusted to give the final volume. The formulations were sterile-filtered and filled in pharmaceutically acceptable containers, such as vials or cartridges.

The results of this experiment are shown in Table 1. This example shows that addition of the preservative meta-cresol in formulation 1 reduced the physical stability of the formulation and led to precipitation of the active drug ZP1848. The example also shows that this effect can be circumvented by lowering the pH of the formulation as shown from the results with formulations at pH 6.0 and pH 5.0.

### Example 3: Stable formulations at pH lower than 7.0

Excipient stock solutions of L-histidine (200 mM), mannitol (500 mM) and m-cresol (1034 mg/ml) were prepared. Excipient solutions were mixed according to the compositions set out in Table 2, and stock solution of ZP1848 (approx. 40 mg/mL) were added. The volume was adjusted to 90% of final volume, pH was adjusted with acetic acid (200 mM) and/or L-arginine (200 to 250 mM), m-cresol was added, and the volume was adjusted to the final volume. The formulations were then sterile-filtered and filled in pharmaceutically acceptable containers, such as vials or cartridges.

The results of this experiment are shown in Table 2. This example confirmed that reducing the pH of the formulation, for example to a pH of less than 7.0, had the effect of improving the physical stability of formulations that include a preservative, reducing the precipitation of the active drug, ZP1848.

### Example 4: Stable formulations containing propylene glycol

Excipient stock solutions of L-histidine (200 mM), mannitol (500 mM), propylene glycol (500 mM) and m-cresol (1034 mg/mL) were prepared. Excipients solutions were mixed according to the desired composition (see Table), and stock solution of ZP1848 (approx. 40 mg/mL) was added. The volume was adjusted to 90% of final volume, the pH was adjusted with acetic acid (1 M) and/or L-arginine (200 or 250 mM), m-cresol was added, and the volume was adjusted to the final volume. The formulations were then sterile-filtered and filled in pharmaceutically acceptable containers, such as cartridges. The composition and physical stability at 5°C of ZP1848 formulations in cartridges was evaluated by examining the visual appearance of the formulation.

The results of this experiment are shown in Table 3.

While the present invention has been described in conjunction with the embodiments described above, many equivalent modifications and variations will be apparent to those skilled in the art when given this disclosure. Accordingly, the embodiments of the invention set forth are considered to be illustrative and not limiting. Various changes to the described embodiments may be made without departing from the spirit and scope of the invention. All documents cited herein are expressly incorporated by reference in their entirety for all purposes.

**Table 1. Effect of m-cresol. Composition and physical stability at 5°C of ZP1848 formulations as evaluated by visual appearance.**

| **Formulation** | **pH** | **ZP1848 (mg/mL)** | **Mannitol (mM)** | **Histidine (mM)** | **Meta-cresol (mg/mL)** | **Other preservative agent** | **Visual appearance (clear/turbid/precipitated)** | | |
|---|---|---|---|---|---|---|---|---|---|
| | | | | | | | **0 weeks** | **5 weeks** | **13 weeks** |
| 1 | 7.0 | 10 | 230 | 15 | - | - | Clear | Clear | Clear |
| 2 | 7.0 | 10 | 230 | 15 | 3.15 | - | Clear | Precipitated | Precipitated |

**Table 2. Effect of lower pH. Composition and physical stability at 5°C of ZP1848 formulations as evaluated by visual appearance.**

| **Formulation** | **pH** | **ZP1848 (mg/mL)** | **Mannitol (mM)** | **Histidine (mM)** | **Meta-cresol (mg/mL)** | **Visual** | | |
|---|---|---|---|---|---|---|---|---|
| | | | | | | **(clear/turbid/precipitated)** | | |
| | | | | | | **0 weeks** | **13 weeks** | **26 weeks** |
| **1** | 7 | 10 | 230 | 15 | 3.15 | Clear | Precipitated | Precipitated |
| **2** | 6.8 | 10 | 230 | 15 | 3.15 | Clear | Clear | Clear |
| **3** | 6.6 | 10 | 230 | 15 | 3.15 | Clear | Clear | Clear |
| **4** | 6.4 | 10 | 230 | 15 | 3.15 | Clear | Clear | Clear |
| **5** | 6.3 | 10 | 230 | 15 | 3.15 | Clear | Clear | Clear |
| **6** | 6.0 | 10 | 230 | 15 | 3.15 | Clear | Clear | Clear |
| **7** | 5.6 | 10 | 230 | 15 | 3.15 | Clear | Clear | Clear |

**Table 3. Effect of propylene glycol. Composition and physical stability at 5°C of ZP1848 formulations as evaluated by visual appearance.**

| **Formulation** | **pH** | **ZP1848 (mg/ml)** | **Mannitol [mM]** | **Histidine [mM]** | **Meta-cresol (mg/mL)** | **Propylene glycol (mM)** | **Visual appearance** | | |
|---|---|---|---|---|---|---|---|---|---|
| | | | | | | | **(clear/turbid)** | | |
| | | | | | | | **0 weeks** | **13 weeks** | **26 weeks** |
| **1** | 7 | 10 | 230 | 15 | 3.15 | - | Clear | Precipitated | Precipitated |
| **2** | 7 | 10 | 230 | 15 | - | - | Clear | Clear | Clear |
| **3** | 7 | 10 | 100 | 15 | 3.15 | 150 | Clear | Clear | Clear |
| **4** | 6.5 | 10 | 100 | 15 | 3.15 | 150 | Clear | Clear | Clear |
| **5** | 6 | 10 | 100 | 15 | 3.15 | 150 | Clear | Clear | Clear |
| **6** | 6 | 10 | 100 | 15 | 3.15 | 150 | Clear | Clear | Clear |
| **7** | 6 | 10 | 100 | 15 | 3.15 | 150 | Clear | Clear | Clear |
| **8** | 5.5 | 10 | 100 | 15 | 3.15 | 150 | Clear | Clear | Clear |
| **9** | 6 | 10 | 230 | 15 | 3.15 | - | Clear | Clear | Clear |
| **10** | 6 | 10 | 200 | 15 | 3.15 | 50 | Clear | Clear | Clear |
| **11** | 6 | 10 | 50 | 15 | 3.15 | 200 | Clear | Clear | Clear |

## Claims

1. A stable liquid pharmaceutical multi-dose formulation, the formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
(a) the GLP-2 analogue at a concentration of about 1 mg/mL to about 30 mg/mL;
(b) meta-cresol as a preservative at a concentration of 1.0 to 5.0 mg/mL;
(c) a histidine buffer at a concentration of about 5 mM to about 50 mM;
(d) a non-ionic tonicity modifier selected from the group consisting of mannitol, sucrose, glycerol and sorbitol at a concentration of about 20 mM to about 360 mM;
(e) a pH of about 5.0 to about 6.8.

2. A stable liquid pharmaceutical multi-dose formulation, the formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt or derivative thereof;
wherein the formulation comprises:
(a) the GLP-2 analogue at a concentration of about 1 mg/mL to about 30 mg/mL;
(b) meta-cresol as a preservative at a concentration of 1.0 to 5.0 mg/mL;
(c) a histidine buffer at a concentration of about 5 mM to about 50 mM;
(d) a non-ionic tonicity modifier selected from the group consisting of mannitol, sucrose, glycerol and sorbitol at a concentration of about 20 mM to about 360 mM;
(e) a pH of about 5.5 to about 7.0; and
(f) propylene glycol at a concentration of 20 mM to 300 mM.

3. The formulation of claim 2, wherein the formulation comprises propylene glycol at a concentration of 20 mM to 300mM.

4. The formulation of claim 2 or claim 3, wherein the formulation comprises propylene glycol at a concentration of 50 mM to 200mM.

5. The formulation according to any one of claims 1 to 4, wherein the formulation is an aqueous formulation.

6. The formulation according to any one of claims 1 to 5, wherein the formulation is stable for at least 18 months when stored at 2-8°C.

7. The formulation according to any one of the preceding claims, wherein the GLP-2 analogue is present in the formulation at a concentration of about 1 mg/mL to about 20 mg/mL.

8. The formulation according to any one of the preceding claims, wherein the GLP-2 analogue is present in the formulation at a concentration of about 2 mg/mL to about 15 mg/mL.

9. The formulation according to any one of the preceding claims, wherein the formulation is a ready-to-use formulation.

10. The formulation according to any one of the preceding claims, wherein the GLP-2 analogue is present in the formulation at a concentration of about 2 mg/mL, 10 mg/mL or 20 mg/mL.

11. The formulation according to any one of the preceding claims, wherein the histidine buffer is present in the formulation at a concentration of about 5 mM to about 25 mM.

12. The formulation according to claim 11, wherein the histidine buffer is present in the formulation at a concentration of about 15 mM.

13. The formulation according to any one of the preceding claims, wherein the mannitol is present in the formulation at a concentration of about 150 mM to about 250 mM.

14. The formulation according to claim 13, wherein the mannitol is present in the formulation at a concentration of about 230 mM.

15. The formulation according to claim 1, wherein the formulation has a pH of about 6.2 to about 6.8.

16. The formulation according to claim 2, wherein the formulation has a pH of about 6.0 to about 7.0.

17. The formulation according to claim 1, wherein the formulation comprises:
(a) the GLP-2 analogue at a concentration of about 2 mg/mL to about 20 mg/mL;
(b) meta-cresol as a preservative at a concentration of 1.0 to 5.0 mg/mL;
(c) a histidine buffer at a concentration of about 5 mM to about 50 mM;
(d) mannitol as a non-ionic tonicity modifier at a concentration of about 20 mM to about 360 mM; and
(e) a pH of about 6.0 to about 6.8.

18. The formulation according to claim 1 or claim 17, wherein the formulation comprises:
(a) the GLP-2 analogue at a concentration of about 1 mg/mL or about 10 mg/mL;
(b) a meta-cresol as a preservative at a concentration of 3.0 to 4.0 mg/mL;
(c) a histidine buffer at a concentration of about 10 mM to about 20 mM;
(d) mannitol as a non-ionic tonicity modifier at a concentration of about 200 mM to about 250 mM; and
(e) a pH of about 6.0 to about 6.8.

19. The formulation according to any one of claims 1, 17 or 18, wherein the formulation consists of the GLP-2 analogue at a concentration of about 2 mg/mL or about 10 mg/mL; a meta-cresol as a preservative at a concentration of 3.0 to 4.0 mg/mL; a histidine buffer at a concentration of about 10 mM to about 20 mM; mannitol as a non-ionic tonicity modifier at a concentration of about 200 mM to about 250 mM; the formulation having a pH of about 6.0 to about 6.8.

20. The formulation according to claim 2, wherein the formulation comprises:
(a) the GLP-2 analogue at a concentration of about 5 mg/mL to about 20 mg/mL;
(b) meta-cresol as a preservative at a concentration of 1.0 to 5.0 mg/mL;
(c) a histidine buffer at a concentration of about 5 mM to about 50 mM;
(d) mannitol as a non-ionic tonicity modifier at a concentration of about 20 mM to about 360 mM;
(e) a pH of about 5.0 to about 7.0; and
(f) propylene glycol at a concentration of 20 mM to 300 mM.

21. The formulation according to claim 2 or claim 20, wherein the formulation comprises:
(a) the GLP-2 analogue at a concentration of about 10 mg/mL;
(b) a meta-cresol as a preservative at a concentration of 3.0 to 4.0 mg/mL;
(c) a histidine buffer at a concentration of about 10 mM to about 20 mM;
(d) mannitol as a non-ionic tonicity modifier at a concentration of about 50 mM to about 230 mM; and
(e) a pH of about 5.0 to about 7.0; and
(f) propylene glycol at a concentration of 50 mM to 200 mM.

22. The formulation according to any one of claims 2, 20 or 21, wherein the formulation consists of the GLP-2 analogue at a concentration of about 10 mg/mL; meta-cresol as a preservative at a concentration of 3.0 to 4.0 mg/mL; histidine buffer at a concentration of about 10 mM to about 20 mM; mannitol as a non-ionic tonicity modifier at a concentration of about 50 mM to about 230 mM; and propylene glycol at a concentration of 50 mM to 200 mM, the formulation having a pH of about 5.0 to about 7.0.

23. The formulation according to any one of the preceding claims, wherein the histidine buffer is L-histidine.

24. The formulation according to any one of the preceding claims, wherein the mannitol is D-mannitol.

25. The formulation according to any one of the preceding claims, wherein the formulation is stable at 2-8°C for at least 6 months, at least 12 months, at least 18 months or at least 24 months.

26. The formulation according to claim 25, wherein the GLP-2 analogue in the formulation retains at least about 90% of its biological activity after 18 months of storage 2-8°C.

27. The formulation according to any one of the preceding claims which is sterile.

28. The formulation according to any one of the preceding claims, wherein the formulation is administration to a subject by injection.

29. The formulation according to claim 28, wherein the injection is subcutaneous injection.

30. The formulation according to any one of the preceding claims, wherein the GLP-2 analogue is provided as an acetate salt.

31. The formulation according to any one of the preceding claims, wherein the GLP-2 analogue is ZP1848 or ZP1848-acetate.

32. An article of manufacture or a kit comprising a container holding the stable pharmaceutical formulation of any one of claims 1 to 31.

33. A delivery device containing a liquid formulation comprising a GLP-2 analogue of any one of claims 1 to 31.

34. The delivery device of claim 33, wherein the delivery device is pre-filled syringe, an injector device, an injector pen, an adjustable dose auto-injector, a disposable auto-injector, a wearable injector, or an infusion pump.

35. A formulation of the glucagon-like peptide 2 (GLP-2) analogue of any one of claims 1 to 31 for use in therapy.

36. A formulation of the glucagon-like peptide 2 (GLP-2) analogue of any one of claims 1 to 31 for use in a method for the treatment and/or prevention of a stomach and/or bowel-related disorder in a human patient.

37. The formulation of the glucagon-like peptide 2 (GLP-2) analogue for use in the method of treatment and/or prevention of claim 36, wherein the stomach and/or bowel-related disorder is ulcers, digestion disorders, malabsorption syndromes, short-gut syndrome, cul-de-sac syndrome, inflammatory bowel disease, celiac sprue (for example arising from gluten induced enteropathy or celiac disease), tropical sprue, hypogammaglobulinemic sprue, enteritis, regional enteritis (Crohn's disease), ulcerative colitis, small intestine damage or short bowel syndrome (SBS).

38. The formulation of the glucagon-like peptide 2 (GLP-2) analogue for use in the method of treatment and/or prevention of claim 37, wherein the stomach and/or bowel-related disorder is short bowel syndrome.

39. The formulation of the glucagon-like peptide 2 (GLP-2) analogue for use in a method for the treatment according to claim 36, wherein the stomach and/or bowel-related disorder is radiation enteritis, infectious or post-infectious enteritis, or small intestinal damage due to toxic or other chemotherapeutic agents.

40. The formulation of the glucagon-like peptide 2 (GLP-2) analogue for use in a method for the treatment according to claim 39, wherein treatment with the GLP-2 analogue is combined with one or more anti-cancer therapies.

41. The formulation of the glucagon-like peptide 2 (GLP-2) analogue for use in a method for the treatment according to claim 40, wherein treatment the anti-cancer therapy comprises administering one or more chemotherapeutic agent(s) to the patient or treating the patient with radiation therapy.

42. The formulation of the glucagon-like peptide 2 (GLP-2) analogue for use in a method for the treatment according to claim 40 or claim 41, wherein the formulation is used in the treatment and/or prevention of a side effect of chemotherapy or radiation treatment.

43. The formulation of the glucagon-like peptide 2 (GLP-2) analogue for use in a method for the treatment according to claim 42, wherein the side effect of chemotherapy is diarrhoea, abdominal cramping, vomiting or structural and functional damage of the intestinal epithelium resulting from chemotherapy treatment.

44. The formulation of the glucagon-like peptide 2 (GLP-2) analogue for use in a method for the treatment according to any one of claims 40 to 43, wherein the human patient is a patient having SBS-intestinal failure.

45. The formulation of the glucagon-like peptide 2 (GLP-2) analogue for use in a method for the treatment according to any one of claims 40 to 44, wherein the human patient is a patient being on the border between being a patient having SBS-intestinal insufficiency and SBS-intestinal failure.

46. The formulation of the glucagon-like peptide 2 (GLP-2) analogue for use in a method for the treatment according to any one of claims 35 to 45, wherein the method comprises administering the GLP-2 analogue to the patient once or twice daily.

47. The formulation of the glucagon-like peptide 2 (GLP-2) analogue for use in a method for the treatment according to any one of claims 29 to 45, wherein the method comprises administering the GLP-2 analogue to the patient once or twice weekly.

48. Use of a formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
for providing an liquid pharmaceutical formulation which is stable for 18 months when stored at 2-8°C, wherein the formulation comprises:
(a) the GLP-2 analogue at a concentration of about 1 mg/mL to about 30 mg/mL;
(b) meta-cresol as a preservative at a concentration of 1.0 to 5.0 mg/mL;
(c) a histidine buffer at a concentration of about 5 mM to about 50 mM;
(d) a non-ionic tonicity modifier selected from the group consisting of mannitol, sucrose, glycerol and sorbitol at a concentration of about 20 mM to about 360 mM;
(e) a pH of about 5.0 to about 6.8.

49. Use of a formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
for providing an liquid pharmaceutical formulation which is stable for 18 months when stored at 2-8°C, wherein the formulation comprises:
wherein the formulation comprises:
(a) the GLP-2 analogue at a concentration of about 1 mg/mL to about 30 mg/mL;
(b) meta-cresol as a preservative at a concentration of 1.0 to 5.0 mg/mL;
(c) a histidine buffer at a concentration of about 5 mM to about 50 mM;
(d) a non-ionic tonicity modifier selected from the group consisting of mannitol, sucrose, glycerol and sorbitol at a concentration of about 20 mM to about 360 mM;
(e) a pH of about 5.5 to about 7.0; and
(f) propylene glycol at a concentration of 20 mM to 300 mM .

50. A process for producing a stable liquid pharmaceutical formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl
X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
wherein the process comprising formulating (a) the GLP-2 analogue at a concentration of about 1 mg/mL to about 30 mg/mL; (b) meta-cresol as a preservative at a concentration of 1.0 to 5.0 mg/mL; (c) a histidine buffer at a concentration of about 5 mM to about 50 mM; (d) a non-ionic tonicity modifier selected from the group consisting of mannitol, sucrose, glycerol and sorbitol at a concentration of about 20 mM to about 360 mM; at a pH of about 5.0 to about 6.8.

51. A process for producing a stable liquid pharmaceutical formulation comprising a glucagon-like peptide 2 (GLP-2) analogue, wherein the GLP-2 analogue is represented by the formula: 
R1-Z1-His-Gly-Glu-Gly-X5-Phe-Ser-Ser-Glu-Leu-X11-Thr-Ile-Leu-Asp-Ala-Leu-Ala-
Ala-Arg-Asp-Phe-Ile-Ala-Trp-Leu-Ile-Ala-Thr-Lys-Ile-Thr-Asp-Z2-R2
 wherein:
R¹ is hydrogen, C₁₋₄ alkyl (e.g. methyl), acetyl, formyl, benzoyl or trifluoroacetyl X5 is Ser or Thr
X11 is Ala or Ser
R² is NH₂ or OH;
Z¹ and Z² are independently absent or a peptide sequence of 1-6 amino acid units of Lys; or a pharmaceutically acceptable salt or derivative thereof;
wherein the process comprising formulating (a) the GLP-2 analogue at a concentration of about 1 mg/mL to about 30 mg/mL; (b) meta-cresol as a preservative at a concentration of 1.0 to 5.0 mg/mL; (c) a histidine buffer at a concentration of about 5 mM to about 50 mM; (d) a non-ionic tonicity modifier selected from the group consisting of mannitol, sucrose, glycerol and sorbitol at a concentration of about 20 mM to about 360 mM; (e) propylene glycol at a concentration of 20 mM to 300 mM ; at a pH of about 5.5 to about 7.0.
